# EUROPEAN PATENT SPECIFICATION

(11) **EP 2 758 984 B1**
(45) Date of publication and mention of the grant of the patent: **15.05.2019**
(21) Application number: 12761567.2
(22) Date of filing: 07.09.2012
(51) Int. Cl.: H01J 61/44, A01K 29/00, A61N 5/06, H01J 61/12

(54) **ANIMAL FARM INCLUDING APPARATUS AND METHOD FOR PROMOTING D-VITAMIN PRODUCTION IN A LIVING ORGANISM**
BAUERNHOF MIT EINER VORRICHTUNG ZUR FÖRDERUNG DER PRODUKTION VON VITAMIN D IN EINEM LEBENDEN ORGANISMUS
FERME D'ÉLEVAGE D'ANIMAUX COMPRENANT UN APPAREIL POUR PROMOUVOIR LA PRODUCTION DE VITAMINE D DANS UN ORGANISME VIVANT

(30) Priority: 21.09.2011 EP 11182204
(43) Date of publication of application: 30.07.2014
(73) Proprietor: SR LIGHT ApS, 7400 Herning (DK)
(72) Inventor: KAAS, Povl, 7400 Herning (DK); JAKOBSEN, Jette, 2860 Søborg (DK)
(74) Representative: Høiberg P/S
(86) International application number: PCT/EP2012/067482
(87) International publication number: WO 2013/041389

(56) References cited:
- EP-A1- 1 970 423
- JP-A- 11 000 046
- US-A- 3 202 811
- US-A1- 2005 007 021

## Description

### Field of invention

The invention relates to an animal farm production facility comprising at least one apparatus for promoting D-vitamin production in a living organism, comprising at least one lamp.

### Background of the Invention

It is known in the art that natural light promotes the production of D-vitamins in the human body.

In EP 1 970 423 A1 there is disclosed a fluorescent lamp for stimulating previtamin D3 production in the human skin. The lamp is a low pressure mercury discharge lamp which has a limited intensity and produces monochromatic light, i.e. just one dominant spectral line.

WO 2009/094100 discloses an enhanced UV-emitting fluorescent lamp that provides a UV spectral emission for simultaneously tanning of the human skin and promotion of vitamin D production in the human body. The disclosed lamp is a monochromatic low pressure lamp. Low pressure lamps only supply monochromatic light (just one dominant spectral line). In addition the power rating does not go beyond a few hundreds watt which is a disadvantage if the lamps are to be installed, for example, in a cowhouse at a distance of 3 to 4 meters from the cows.

WO2010/102039 discloses a method for increasing the vitamin D content of a mushroom by exposing the mushroom to a lamp that emits UVA and UVB, but not UVC radiation. It is noted that the mushrooms are only exposed to UVA and UVB radiation but not natural light. In addition, it is noted that there is an increase in the content of D2 vitamin and for example not of the other important D vitamins, such as the D3 vitamin.

It is known that when an animal, such as a cow, kept outside in a field in the summer where it is exposed to natural sunlight its D-vitamin status is increased and the content of D vitamin in its milk is accordingly increased. As modern farming involves keeping the livestock indoor inside an animal farm production facility the natural production of D vitamin is compromised. To compensate for this addition of D-vitamin to the food is used. However, it is known that the level of D-vitamin obtainable by exposing a human or an animal to sunlight is much higher than the level obtainable through food additives. Moreover, it has been discovered that the skin of a human or an animal is a much more efficient in producing D-vitamin if exposed to sunlight and then it is not possible to achieve a toxic dozes of D-vitamin when exposed to the sun.

Considering the prior art described above, it is an object of the present invention to provide an animal farm production facility comprising at least one apparatus. In particular, it is an object to increase the production of D3 vitamin in a livestock, such as cattle, pigs, chickens and the like.

### Summary of the invention

The object is achieved by an apparatus for promoting D-vitamin production in a living organism, comprising at least one lamp assembly, said at least one lamp assembly is adapted to emit polychromatic light, wherein the polychromatic light at least emulates natural light and UV light at wavelengths between 270 nm and 315 nm, wherein the at least one lamp assembly comprises at least one medium and/or high pressure lamp.

By providing a combination of natural light and UV light at wavelengths between 270 nm and 315 nm, the production of D-vitamin in living organisms is enhanced. There is specifically the advantage in relation to the dairy farming industry that in milk producing mammals, such as cows, the D-vitamin content of the milk is increased. Especially, it has been found that the vitamin D3 content is increased, and especially that the D-vitamin content in the milk from cows is increased when exposed to polychromatic light comprising both natural light and light in at wavelengths between 270 nm and 315 nm.

Natural light is to be understood as sunlight as it appears on the earth surface during day within the normal variations in intensity and spectrum. The spectrum and intensity of sunlight varies depending on the atmosphere, ozone layer and the position of the sun, and here natural light refer to any sunlight within those normal variations.

Polychromatic light is to be understood as light exhibiting a plurality of colours, in other words that the light comprises a plurality of wavelengths.

In an embodiment, the polychromatic light appears to the naked eye to be continuous, such as polychromatic light at a frequency over 50Hz. The use of a flashing or pulsing light will stress the living organism. Therefore, it is an advantage to ensure that the light appears to be continuous to the living organism, such as an animal. This can be ensured by having a frequency above 50Hz, 100Hz, 200Hz, 500Hz, 1000Hz, 2000Hz or 3000Hz.

Preferably, the apparatus comprises means for removing light of a wavelength under 270 nm. There is health risks involved with exposure to light at a wavelength under 270nm. Therefore, living organisms, such as mammals e.g. cows, can be protected by removing the harming light. The means for removing light with a wavelength under 270nm can be a filter that is transparent to light of a wavelength above 270 and opaque for wavelength under 270nm.

Advantageously, said at least one lamp assembly comprises at least one medium pressure lamp and/or high pressure lamp. When using a medium pressure lamp and/or high pressure lamp, it is possible to reach higher power compared to low pressure lamps. This is especially advantageously when installing the apparatus in an animal farm producing facility, such as a stable, cowhouse or pigsty or any other larger building, where the apparatus supplies light to more than one animal. In that case, the apparatus can be positioned at a distance of 2, 3 or 4 or more meters away from the animals, and in order to supply sufficient light to the animals, a high power is preferred. A medium pressure lamp is to be understood as a lamp with a gas pressure of 0.01 to 1 MPa. A high pressure lamp is to be understood as a lamp with a gas pressure of 3 to 10 MPa. A low pressure lamp is to be understood as a lamp with a gas pressure of 0.01 to 133 Pa.

In an embodiment, said at least one lamp assembly comprises a first lamp emulating natural light and a second lamp providing UV light at wavelengths between 270 nm and 315 nm. In this embodiment, the first lamp can be any lamp that emulates natural light, such as a sulphur lamp, and the second lamp can be a fluorescent lamp or a medium pressure lamp, preferably a medium pressure lamp with enhanced energies at wavelength between 270nm and 315nm. There can be any number of lamps in order to secure that the area covered by the emitted light is as extensive as possible.

In an embodiment, said at least one lamp assembly comprises a doped lamp, preferably a doped sulphur lamp, a mercury lamp and/or a xenon lamp, which emits natural light in conjunction with UV light. In this embodiment, it is possible to use only one lamp as it can provide the full spectrum needed.

Advantageously, the doped lamp is a sulphur lamp, a mercury lamp and/or a xenon lamp, doped with a metal, non-metal or metalloids, preferably tin, lead, iron, phosphorus, sulphur, selenium, boron, silicon, germanium, arsenic, antimony and /or tellurium. Doping the lamp with metal and especially with the mentioned metals will enhance the intensities of the UV light at wavelengths between 270 nm and 315 nm.

A further aspect is the use of an apparatus as described herein to increase D-vitamin production in animals, such as cows. The apparatus as described above can be used to enhance the D-vitamin production within the living organism of an animal. Thus, the D-vitamin content in the meat form the animals exposed to the apparatus described above. This can prevent humans eating the meat from suffering from vitamin D deficiency. These animals can be birds, such as chickens and/or mammals, such as cows, pigs, goats and/or lambs.

An apparatus as described can be used to increase D-vitamin content in milk from a mammal, preferably a cow. Increasing the vitamin D content in milk by using of the present invention has the advantage that humans consuming the milk can prevent vitamin D deficiency. In parts of the earth away from equator, the daytime light can be limited during winter. This is, for example, the case in northern Europe during the months November to March. During that time, the lack of exposure to the sun can cause people to suffer from vitamin D deficiency. In any case the sun is only important if the cows are outside. In Northern Europe, and other industrialised countries at the same latitude north or south, the majority of milk cows are inside a cowhouse and as a consequence do not produce D vitamins in their milk unless the animals are exposed to the polychromatic light produced by an apparatus according to the first aspect of the present invention. Thus, by the invention a compensation for the lack of natural sunlight is provided, whereby the produced milk is naturally produced with a D-vitamin content also at winter and thereby help preventing the lack of vitamin D in the human body of the people drinking the milk. This is particularly advantageous in relation to organic farming where artificial nutritious food supplements are not allowed. By the present invention, the D-vitamin content is kept at a high level without any dietary supplements to the animal food whereby the animals are farmed organically.

An aspect of the present invention is an animal farm production facility comprising at least one apparatus according to the present invention. As the apparatus emits both visible light and UV light, an animal farm production facility with the apparatus of the present invention does not need any additional lightening system. The present invention can substitute the conventional illumination of the cow house which saves money at installation as only one illumination system is needed.

In accordance with this aspect, an animal farm production facility, wherein one or more livestock are accommodated for production of one or more farm products, might involve the lighting apparatus throughout the entire building or it could be one or more lamps in the ceiling lighting amongst traditional lighting facilities. According to the invention, the animal farm production facility could also include one or more cows which are accommodated for production of milk, where the light source is provided in relation to a milking station or the like.

In general, it is realised that the effect of increasing the D-vitamin will benefit the health of the livestock. The animals will thereby achieve a better immune defence system, whereby less or no antibiotics is needed in the animal farming production, just as a better animal welfare is achieved and less environmental impact, just as a natural D-vitamin source is provided for humans when utilising the invention in an animal farm production.

By the invention, it is also realised that the using the apparatus in an animal farm production is beneficial to birds, such as chickens or hens, for production of eggs. The promotion of D-vitamin in the eggs increases the nutritional value of the eggs as a human food source.

As a further aspect of the invention, there is provided a method of increasing D-vitamin production in an animal, by exposing the animal to polychromatic light, wherein the polychromatic light at least emulates natural light and UV light at wavelengths between 270 nm and 315 nm. In particular, the invention also concerns a method for increasing D-vitamin content in milk, preferably cow milk, by exposing an animal, preferably cow, producing said milk to polychromatic light, wherein the polychromatic light at least emulates natural light and UV light at wavelengths between 270 nm and 315 nm. By using the method, it is possible to increase vitamin D content in milk which is beneficial to the milk consumer. Lack of vitamin D can lead to health problems, such as impaired bone mineralization, and bone softening diseases. Those deficiencies can be avoided if milk with increased vitamin D content is consumed.

Advantageously, the polychromatic light appears to the naked eye to be continuous, such as polychromatic light with a frequency over 50Hz. In order to avoid stressing the animals, the light should appear to be continuous to the animal. A stressed animal can result in a decreased milk production and is therefore not desirable. The frequency can for example be above 50Hz, 100Hz, 200Hz, 500Hz, 1000Hz, 2000Hz or 3000Hz.

Preferably, the animal is only exposed to light at a wavelength over 270 nm. Light in the UV range can be harmful to living organisms. Therefore, it is desirable to limit the exposure of the animals to light at a wavelength under 270. This can for example be achieved by using a filter which remove light under the desired wavelength and/or designing the light emitter in such a way that no light under 270nm is produced.

In an embodiment, polychromatic light is provided by a plurality of lamps. This can make the installation easier and in addition it is possible to turn off only some of the lamps. This can be advantageous, if for example a first lamp for natural light and a second lamp for the light at wavelengths between 270 nm and 315 nm are used. Then the light having wavelengths between 270 nm and 315 nm can be turned on for only a limited time for example 2 to 3 hours every day and the natural light can be on the entire day and be used as the primary illumination of the building.

It is found that in order for the animal to promote a satisfactory D-vitamin production, the animal needs only to be exposed for a predetermined amount of time daily, such as 30 minutes daily, 60 minutes daily, 90 minutes daily or 120 minutes daily.

### Description of the drawings

The invention will in the following be described in greater detail with reference to the accompanying drawings, in which:
- Fig. 1: shows a graph of a spectrum for a doped mercury UV lamp;
- Fig. 2: shows a graph of a spectrum for sulphur lamps;
- Fig. 3A and 3B: show a schematic side and top view - respectively - of a lamp assembly according to an embodiment of the present invention;
- Fig. 4: shows a schematic front view of a lamp assembly according to an embodiment of the present invention;
- Fig. 5: shows a graph showing test results;
- Fig. 6: shows a comparative test of D3 vitamin contents in pigs; and
- Fig. 7: shows a comparative test between natural exposure and exposure to lightning from a lamp according to the invention.

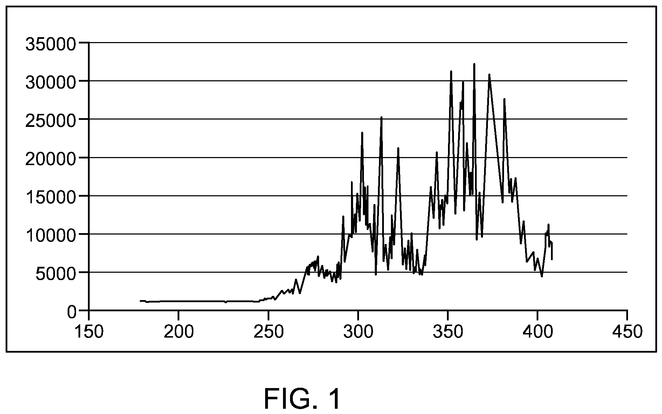

Fig. 1 shows intensities as a function of wavelength of a doped mercury UV lamp. As can be seen, there is light in the range from approximately 250 nm and up. A line is shown at 270 nm because a filter can be installed in order to remove light under that wavelength.

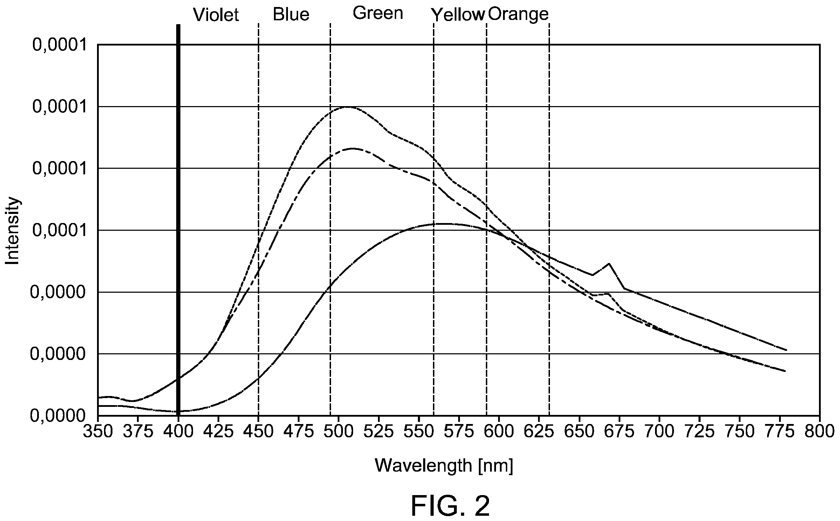

Fig. 2 shows a graph for sulphur lamps of different powers. The spectrum is very similar to daytime solar light at the earth's surface. Therefore, a sulphur lamp can be used to provide natural light. Sulphur lamps are known in the art and will not be described in detail. As can be seen, there is little UV light. UV light is electromagnetic radiation with a wavelength of between 10nm and 400nm. A sulphur lamp can be doped with metals in order to achieve light emission as close to natural light as technically possible.

A lamp assembly used in the present invention can be a combination of the doped UV lamp and a mercury lamp, as an alternative the sulphur lamp described above can be used. If the lamp assembly comprises these two lamps, it is possible to regulate the UV and the natural light independently. As an example, such a lamp assembly can be installed in an animal farm production building, then the mercury lamp can be used as illumination to represent the daytime and so that the farmer can work in the animal farm production facility, such as a building accommodating cows. The UV lamp can then be turned on and off for only a limited amount of time each day, for example 1, 2 or 4 hours every day. By using the natural light lamp (here exemplified as a mercury lamp) as illumination in the building no other lighting installation is needed. Hence, the costs of the normal illumination can be reduced.

In another embodiment the lamp assembly comprises one doped mercury lamp. The lamp can be doped with, for example Iron, tin, lead, phosphorus, sulphur, selenium, boron, silicon, germanium, arsenic, antimony and /or tellurium. Such a lamp both emulates natural light and has enhanced intensities in the UV area. The light with wavelength under 270 nm can then removed by use of a filter. When using such a lamp there is used only one lamp needs to be installed as it can be used as normal illumination. In addition it will enhance the vitamin D production in living organisms exposed to its light. This means that a farmer only needs to install this type of lamps in his livestock accommodating production facilities in order to increase the vitamin D content of the produced milk.

The following table includes examples of different lamp types that can be used in a lamp assembly.

| **Lamp type** | **no.** | **Min. nm** | **Max. nm** |
|---|---|---|---|
| Mercury florescent low pressure | 1 | 270 | 315 |
| Mercury medium pressure doped | 2 | 270 | 315 |
| Mercury medium pressure doped | 3 | 270 | 900 |
| Mercury medium pressure | 4 | 380 | 900 |
| Mercury high pressure | 5 | 260 | 900 |
| Xenon high pressure | 6 | 300 | 900 |
| Mercury high pressure doped | 7 | 270 | 900 |
| Xenon high pressure doped | 8 | 270 | 900 |
| Sulphur | 9 | 380 | 900 |
| Plasma and/or incandescent lamp. (Solar Simulator) | 10 | 380 | 900 |
| Doped Plasma and/or doped incandescent lamp; (Solar Simulator) | 11 | 270 | 900 |

With use of examples of the lamps in the table different embodiments of a lamp assembly can be made.

The following table has examples of lamp assemblies made from the lamp types from the above shown table.

| **Lamp and combinations of lamps** | **no.** | **no.** |
|---|---|---|
| Lamp assembly A | 1 | 4 |
| Lamp assembly B | 1 | 5 |
| Lamp assembly C | 1 | 6 |
| Lamp assembly D | 1 | 9 |
| Lamp assembly E | 1 | 10 |
| Lamp assembly F | 2 | 4 |
| Lamp assembly G | 2 | 5 |
| Lamp assembly H | 2 | 6 |
| Lamp assembly I | 2 | 9 |
| Lamp assembly J | 2 | 10 |
| Lamp assembly K | 3 | |
| Lamp assembly L | 5 | |
| Lamp assembly M | 7 | |
| Lamp assembly N | 8 | |
| Lamp assembly O | 11 | |

Thus, the lamp assemblies A to J are a combination of two lamp types and the lamp assemblies K to O only use one lamp.

As the lamp assembly is to be used as lighting, it is preferred that the lighting appears to be continuous. A pulsed lamp will not only stress the animals but also make it difficult for the farmer to work, most of the known UV and natural light lamps are to some extend pulsed but as long as the pulses are higher than 50Hz it will appear to both animals and humans to be continuous.

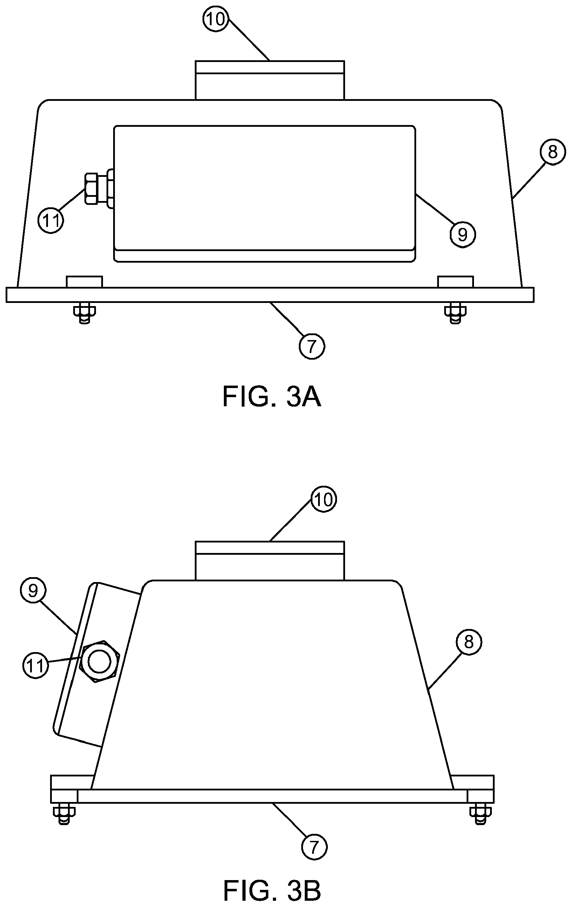

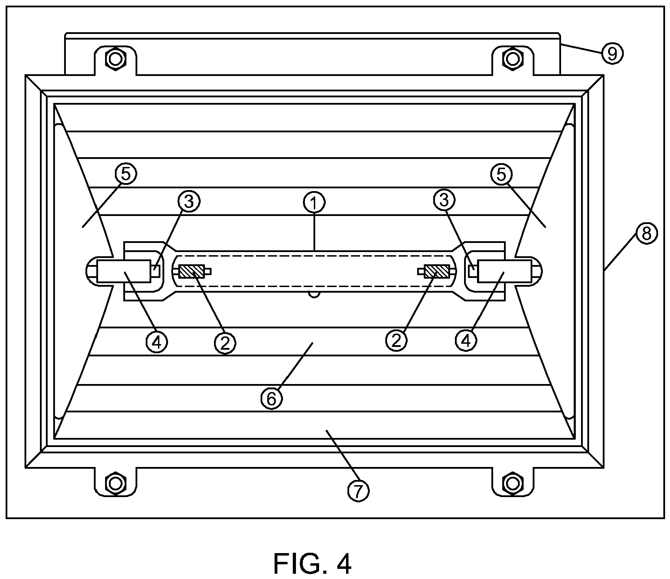

The figures 3A, 3B and 4 show a lamp assembly according to an embodiment of the invention, where a medium and/or high pressure lamp 1 is accommodated in a lamp house 8. The lamp 1 has a set of electrodes 2 at each end which are fitted to electrical power supplies 3 and mounted with electrical insulation sockets 4 in the lamp house 8. The lamp house 8 has side reflectors 5 on each side of the lamp 1, a reflector 6 curved behind the lamp 1, and with an optical edge filter 7 in front of the lamp 1. Outside the reflector 6 on the lamp house 8 an electronic control box 9 is provided with cable connections 11 provided thereon. On the rear of the outside of the lamp house 8 a ventilator 10 is provided whereby the temperature of the lamp assembly may be controlled.

In an embodiment, the lamp assembly has a lamp that can provide both natural light and UV light at the same time. Such a lamp can be a mercury, sulphur, xenon lamp doped with metal, non-metals or metalloids, for example, any combination of tin (Sn), lead (Pb), iron (Fe), phosphorus (P), sulphur (S), selenium (Se), boron (B), silicon (Si), germanium (Ge), arsenic (As), antimony (Sb) and /or tellurium (Te).. These elements can enhance the intensities in the 270nm to 315 nm range.

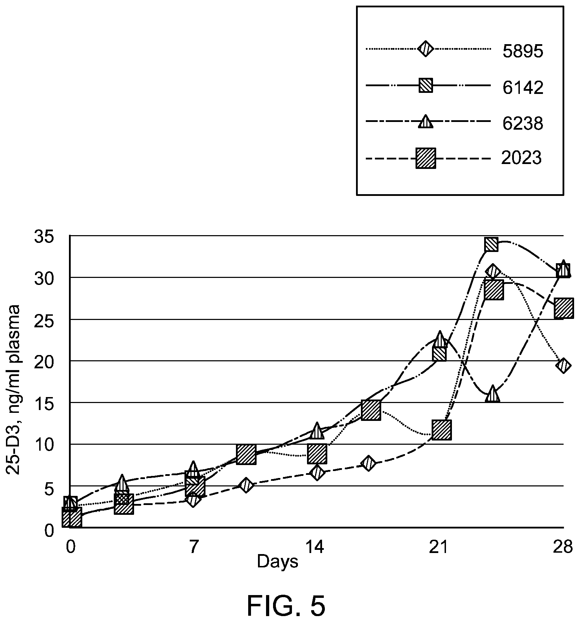

A test of an embodiment of the invention has been performed. Here, the UV lamp having the spectrum as shown in fig. 1 was used together with a sulphur lamp to illuminate cows. The lamp assembly was installed 3 to 3.5 meters from the cows in the test. The lamp was turned on for 30 minutes every 24 hours. This was repeated for 28 days. The test was performed on four cows, having the numbers: 5895, 6142, 6238 and 2023. The cows were milked every day and the vitamin D3 content of the milk was measured for each of the four cows. The result of the test is shown in fig. 5. It can be seen that the content of D3 vitamin increases from about 3 ng/ml to about 25 ng/ml. This is a substantial increase in the D vitamin content of the milk.

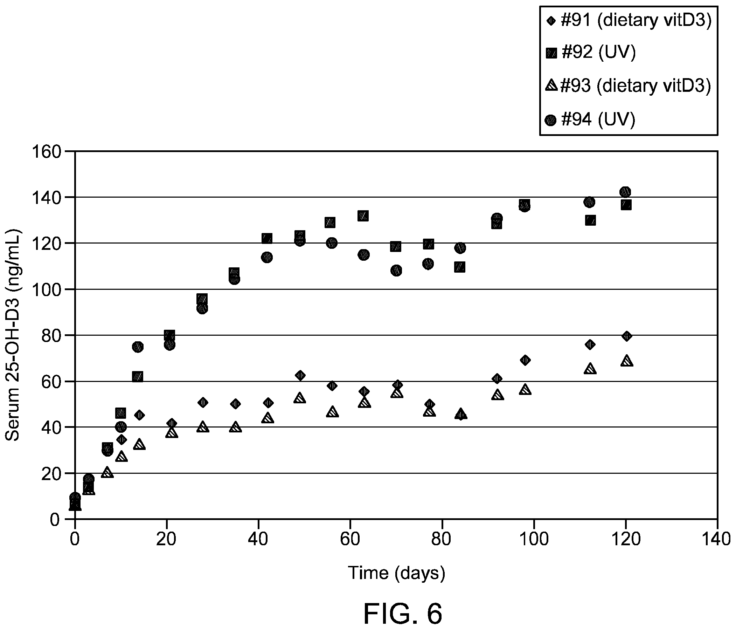

Besides increasing the content of D3 vitamin in the produced milk in cattle farming, it is realised that the UV lamp arrangement according to the invention may also increase the D vitamin content in other animals. As shown in fig. 6, a study of D vitamin status in pigs clearly shows an increase in the content of D vitamin when the pigs are exposed to the UV lamp arrangement according to the invention (lots #92 and #94) compared to the animals given D3 vitamin in their food (lots #91 and #93).

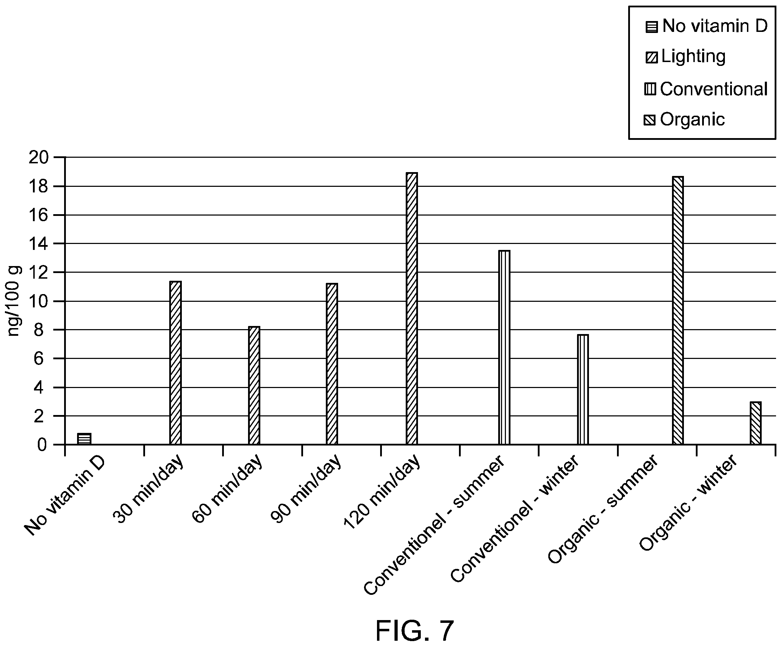

Fig. 7 shows the D3 vitamin content in the produced milk of cows - both conventional cattle and organic bread cattle - when exposed to natural sunlight during the winter and during the summer in comparison with an exposure to the lighting of the UV lamp arrangement according to the invention for a predetermined amount of time every day. As shown in fig. 7, studies also reveal that it is sufficient to expose the animals, such as cows, for 30 minutes every day in order to achieve the desired result to reproduce the D3 vitamin content naturally occurring during summer when the animals are free-ranging outside and thereby exposed to sunlight. In particular these studies reveal that by the present invention it is possible to reproduce the same high amount of D3 vitamin content in milk as in the milk from organically farmed cows during summer but all around the year.

## Claims

1. An animal farm production facility for at least one animal, said facility comprising at least one apparatus which is used to promoting D-vitamin production in a non-human animal, said apparatus comprising at least one lamp assembly, said at least one lamp assembly is adapted to emit polychromatic light, wherein the polychromatic light at least emulates natural light and UV light at wavelengths between 270 nm and 315 nm, wherein the at least one lamp assembly comprises at least one medium and/or high pressure lamp.

2. An animal farm production facility according to claim 1, wherein the polychromatic light appears to the naked eye to be continuous, polychromatic light at a frequency over 50Hz.

3. An animal farm production facility according to any of the preceding claims, wherein the apparatus comprises means for removing light of a wavelength under 270 nm.

4. An animal farm production facility according to any of the preceding claims, wherein said at least one lamp assembly comprises at least one first lamp emulating natural light and the least one medium and/or high pressure lamp providing UV light at wavelengths between 270 nm and 315 nm.

5. An animal farm production facility according to any of the preceding claims, wherein said at least one lamp assembly is selected from a group consisting of one or more of: a doped lamp, a doped sulphur lamp, a mercury lamp and a xenon lamp, which emits natural light in conjunction with UV light.

6. An animal farm production facility according to claim 5, wherein the doped lamp is a sulphur lamp, a mercury lamp and/or a xenon lamp, doped with a metal, non-metal or metalloids.

7. An animal farm production facility according to any of the preceding claims, which is used to increase the content of D-vitamin in the milk produced by said at least one animal.

8. An animal farm production facility according to any of the preceding claims, wherein said at least one animal is accommodated for production of one or more farm products.

9. An animal farm production facility according to any of the preceding claims, wherein one or more cows are accommodated for production of milk.

10. An animal farm production facility according to any of the preceding claims, wherein one or more birds are accommodated for production of eggs.

11. An animal farm production facility according to any of the preceding claims, wherein one or more pigs are accommodated.

12. An animal farm production facility according to claim 1, wherein the at least one animal is exposed with polychromatic light for a predetermined amount of time daily in order to increase the content of D-vitamin in the milk produced by said at least one animal.

## Patentansprüche

1. Produktionsanlage für einen Tierhaltungsbetrieb für mindestens ein Tier, wobei die Anlage mindestens eine Vorrichtung umfasst, die zur Förderung der D-Vitaminproduktion bei einem nichtmenschlichen Tier verwendet wird, wobei die Vorrichtung mindestens eine Lampenanordnung umfasst, wobei die mindestens eine Lampenanordnung zur Emission von polychromatischem Licht ausgelegt ist, wobei das polychromatische Licht mindestens natürliches Licht und UV-Licht bei Wellenlängen zwischen 270 nm und 315 nm emuliert, wobei die mindestens eine Lampenanordnung mindestens eine Mittel- und/oder Hochdrucklampe umfasst.

2. Produktionsanlage für einen Tierhaltungsbetrieb nach Anspruch 1, wobei das polychromatische Licht dem bloßen Auge als kontinuierliches, polychromatisches Licht mit einer Frequenz über 50 Hz erscheint.

3. Produktionsanlage für einen Tierhaltungsbetrieb nach einem der vorhergehenden Ansprüche, wobei die Vorrichtung Mittel zum Entfernen von Licht mit einer Wellenlänge unter 270 nm umfasst.

4. Produktionsanlage für einen Tierhaltungsbetrieb nach einem der vorhergehenden Ansprüche, wobei die mindestens eine Lampenanordnung mindestens eine erste Lampe, die natürliches Licht emuliert, und die mindestens eine Mittel- und/oder Hochdrucklampe, die UV-Licht bei Wellenlängen zwischen 270 nm und 315 nm bereitstellt, umfasst.

5. Produktionsanlage für einen Tierhaltungsbetrieb nach einem der vorhergehenden Ansprüche, wobei die mindestens eine Lampenanordnung ausgewählt wird aus einer Gruppe bestehend aus einer oder mehreren von: einer dotierten Lampe, einer dotierten Schwefellampe, einer Quecksilberlampe und einer Xenonlampe, die natürliches Licht in Verbindung mit UV-Licht emittiert.

6. Produktionsanlage für einen Tierhaltungsbetrieb nach Anspruch 5, wobei die dotierte Lampe eine Schwefellampe, eine Quecksilberlampe und/oder eine Xenonlampe ist, dotiert mit einem Metall, Nichtmetall oder Metalloiden.

7. Produktionsanlage für einen Tierhaltungsbetrieb nach einem der vorstehenden Ansprüche, die verwendet wird, um den Gehalt an D-Vitamin in der von dem mindestens einen Tier produzierten Milch zu erhöhen.

8. Produktionsanlage für einen Tierhaltungsbetrieb nach einem der vorhergehenden Ansprüche, wobei das mindestens eine Tier zur Herstellung eines oder mehrerer landwirtschaftlicher Produkte untergebracht ist.

9. Produktionsanlage für einen Tierhaltungsbetrieb nach einem der vorstehenden Ansprüche, wobei eine oder mehrere Kühe zur Produktion von Milch untergebracht sind.

10. Produktionsanlage für einen Tierhaltungsbetrieb nach einem der vorhergehenden Ansprüche, wobei ein oder mehrere Vögel zur Produktion von Eiern untergebracht sind.

11. Produktionsanlage für einen Tierhaltungsbetrieb nach einem der vorstehenden Ansprüche, wobei ein oder mehrere Schweine untergebracht sind.

12. Produktionsanlage für einen Tierhaltungsbetrieb nach Anspruch 1, wobei das mindestens eine Tier täglich für eine vorbestimmte Zeitdauer polychromatischem Licht ausgesetzt wird, um den Gehalt an D-Vitamin in der von dem mindestens einen Tier produzierten Milch zu erhöhen.

## Revendications

1. Installation de production de ferme d'élevage d'animaux pour au moins un animal, ladite installation comprenant au moins un appareil qui est utilisé pour favoriser la production de vitamine D chez un animal non humain, ledit appareil comprenant au moins un ensemble lampe, ledit au moins un ensemble lampe est adapté pour émettre une lumière polychromatique, dans laquelle la lumière polychromatique au moins imite une lumière naturelle et une lumière UV à des longueurs d'onde situées entre 270 nm et 315 nm, dans laquelle l'au moins un ensemble lampe comprend au moins une lampe à pression moyenne et/ou élevée.

2. Installation de production de ferme d'élevage d'animaux selon la revendication 1, dans laquelle la lumière polychromatique apparaît à l'oeil nu comme une lumière polychromatique continue à une fréquence de plus de 50 Hz.

3. Installation de production de ferme d'élevage d'animaux selon l'une quelconque des revendications précédentes, dans laquelle l'appareil comprend un moyen pour éliminer une lumière d'une longueur d'onde sous 270 nm.

4. Installation de production de ferme d'élevage d'animaux selon l'une quelconque des revendications précédentes, dans laquelle ledit au moins un ensemble lampe comprend au moins une première lampe imitant une lumière naturelle et l'au moins une lampe à pression moyenne et/ou élevée fournissant une lumière UV à des longueurs d'onde situées entre 270 nm et 315 nm.

5. Installation de production de ferme d'élevage d'animaux selon l'une quelconque des revendications précédentes, dans laquelle ledit au moins un ensemble lampe est sélectionné dans un groupe constitué d'un ou de plusieurs parmi : une lampe dopée, une lampe au soufre dopée, une lampe au mercure et une lampe au xénon, qui émet une lumière naturelle conjointement avec une lumière UV.

6. Installation de production de ferme d'élevage d'animaux selon la revendication 5, dans laquelle la lampe dopée est une lampe au soufre, une lampe au mercure et/ou une lampe au xénon, dopée avec un métal, un non-métal ou des métalloïdes.

7. Installation de production de ferme d'élevage d'animaux selon l'une quelconque des revendications précédentes, qui est utilisée pour augmenter la teneur en vitamine D dans le lait produit par ledit au moins un animal.

8. Installation de production de ferme d'élevage d'animaux selon l'une quelconque des revendications précédentes, dans laquelle ledit au moins un animal est logé pour la production d'un ou de plusieurs produits agricoles.

9. Installation de production de ferme d'élevage d'animaux selon l'une quelconque des revendications précédentes, dans laquelle une ou plusieurs vaches sont logées pour la production de lait.

10. Installation de production de ferme d'élevage d'animaux selon l'une quelconque des revendications précédentes, dans laquelle un ou plusieurs oiseaux sont logés pour la production d'oeufs.

11. Installation de production de ferme d'élevage d'animaux selon l'une quelconque des revendications précédentes, dans laquelle un ou plusieurs cochons sont logés.

12. Installation de production de ferme d'élevage d'animaux selon la revendication 1, dans laquelle l'au moins un animal est exposé à une lumière polychromatique pendant une quantité prédéterminée de temps chaque jour afin d'augmenter la teneur en vitamine D dans le lait produit par ledit au moins un animal.
